# EUROPEAN PATENT SPECIFICATION

(11) **EP 2 498 664 B1**
(45) Date of publication and mention of the grant of the patent: **24.01.2018**
(21) Application number: 09851310.4
(22) Date of filing: 24.12.2009
(51) Int. Cl.: A61B 1/00, A61B 1/045, A61B 34/32, A61B 1/04, A61B 34/00, A61B 17/00, A61B 34/30

(54) **CAPSULE TYPE MICRO-ROBOT BIDIRECTIONAL MOVING SYSTEM**
KAPSELARTIGES MIKROROBOTISCHES BIDIREKTIONALES BEWEGUNGSSYSTEM
SYSTÈME MOBILE BIDIRECTIONNEL DE MICRO-ROBOT DE TYPE CAPSULE

(30) Priority: 13.11.2009 KR 20090109720
(43) Date of publication of application: 19.09.2012
(73) Proprietor: Korea Institute of Science and Technology, Seoul 136-791 (KR)
(72) Inventor: YOON, Eui Sung, Seoul 139-939 (KR); YANG, Sung Wook, Gimpo-si Gyeonggi-do 415-778 (KR); KIM, Jinseok, Seoul 156-776 (KR); NA, Kyounghwan, Uiwang-si Gyeonggi-do 437-080 (KR); RHO, Duk Moon, Seoul 132-762 (KR); LEE, Seung Seok, Seoul 136-130 (KR)
(74) Representative: advotec.
(86) International application number: PCT/KR2009/007773
(87) International publication number: WO 2011/059133

(56) References cited:
- JP-A- 2009 018 068
- KR-A- 20030 023 806
- KR-A- 20040 102 927
- KR-B1- 100 618 119
- KR-B1- 100 839 825
- SU-A1- 1 169 765
- US-A1- 2006 257 234
- US-A1- 2007 156 211
- US-B2- 6 824 510

## Description

### Technical Field

The disclosure relates to a capsule type micro-robot bidirectional moving system, more particularly to a capsule type micro-robot bidirectional moving system capable of moving forward and backward in the inside of internal organs.

### Background Art

A method using an endoscope is used as a method for noninvasively treating or examining internal organs in vivo. However, such a typical endoscopic method requires a highly skilled operating technique and gives patients pain.

Therefore, technologies for an in-vivo movement system have been studied, which is inserted into a patient's body through its mouth or anus to treat or examine internal organs without giving the patient pain. A capsule type endoscope that is inserted into a human body through its mouth to photograph the inside of the human body is used as a representative in-vivo movement system.

The capsule type endoscope consists of a micro-miniature camera for photographing in-vivo images, a communication device for transmitting the acquired images to the exterior thereof, and the like. Through the capsule type endoscope, it is possible to photograph the inside of internal organs.

However, since such a capsule type endoscope passively moves in an internal organ, depending upon the peristalsis of the internal organ, its moving speed is entirely slow in the internal organ and remarkably changed depending on characteristics of a digestive organ or patients subjected to operation. Further, since the position of the capsule type endoscope inside organs and the posture of itself are not appropriately controlled depending on conditions, it is difficult to make an accurate diagnosis.

Therefore, studies have been conducted to develop a capsule type micro-robot system that provides a driving means of its own to actively move in the inside of internal organs. However, a current capsule type micro-robot unidirectional moving system possibly moves only in one direction.

Such a capsule type micro-robot unidirectional moving system has difficulty in moving backward to perform a desired operation or photographing process when it passes by parts which need to be diagnosed. A capsule type micro-robot bidirectional moving system has been developed to solve such a problem. However, the structure of the system is complicated, and it is difficult to control the system. Document US2006257234 A1 discloses a capsule type micro-robot moving system having a linearly forward and backward moving selective moving body, driving means for moving said selective moving body, a first moving body, and first and second legs.

### Disclosure of Invention

### Technical Problem

Disclosed herein is a capsule type micro-robot bidirectional moving system which can be easily controlled with a simple structure and move forward and backward in the inside of internal organs.

### Solution to Problem

The invention is defined by independent claim 1. Preferred embodiments are defined in the dependent claims. In an aspect, there is provided a capsule type micro-robot bidirectional moving system including: a selective moving body for moving forward and backward in a straight line along a predetermined axis; a driving means for moving the selective moving body; a first moving body disposed at the front of the selective moving body; a second moving body disposed at the rear of the selective moving body; first legs coupled to the first moving body so as to be folded or unfolded; and second legs coupled to the second moving body so as to be folded or unfolded. The selective moving body may be selectively coupled to or decoupled from the first or the second moving body.

When the system moves forward, the selective moving body may be coupled to the first moving body to allow the first moving body to move in a straight line along the axis. When the system moves backward, the selective moving body may be coupled to the second moving body to allow the second moving body to move in a straight line along the axis.

The selective moving body may include a first latching pin formed to extend to the front thereof and a second latching pin formed to extend to the rear thereof. Pin latching grooves having the first and the second latching pins are coupled or decoupled thereto or therefrom may be formed at the first and the second moving bodies, respectively.

The pin latching groove may include a coupling portion having the latching pin mounted thereon, an entry portion for forming an entry path to the coupling portion, and an exit portion for forming an exit path from the coupling portion. The latching pin may be coupled to the pin latching groove by being entered into the entry portion and mounted on the coupling portion, and the latching pin mounted on the coupling portion may be decoupled from the pin latching groove by being exited through the exit portion.

Steps may be formed at the boundaries between the entry portion, the coupling portion and the exit portion, respectively.

The entry portion may include a first entry path for forming a path through which the latching pin enters into the pin latching groove and a second entry path for forming a path through which the latching pin enters into the coupling portion. The exit portion may include a first exit path for forming a path through which the latching pin is decoupled from the coupling portion and a second exit path for forming a path through which the latching pin is decoupled from the pin latching groove.

Steps may be formed at the boundary between the first and the second entry paths and at the boundary between the first and the second exit paths, respectively.

The selective moving body may include a first elastic body formed at the front thereof and a second elastic body formed at the rear thereof.

The first and the second elastic bodies may be coil type springs.

The system may further include a capsule for accommodating the selective moving body, the first moving body and the second moving body therein. The capsule may be provided with slits formed in the length direction thereof. Each of the first and the second legs may be respectively exposed to the exterior of the capsule through the slits when they are unfolded, and each of the first and the second legs may be respectively accommodated in the interior of the capsule through the slits when they are folded.

The driving means may include a shaft formed in the length direction of the capsule to be rotated by the motor. Hollow portions may be formed at the center of the selective moving body, the first moving body and the second moving body so as to pass through them, respectively. The shaft may be coupled to the selective moving body, the first moving body and the second moving body by passing through them.

A screw thread may be formed on the outer circumferential surface of the shaft. A screw thread engaged with the screw thread of the shaft may be formed on the inner circumferential surface of the hollow portion in the selective moving body. The shaft and the selective moving body may be screw-engaged with each other.

A first key may be formed in the length direction of the capsule on an outer circumferential surface of the selective moving body, and a key way engaged with the first key may be formed on an inner circumferential surface of the capsule for anti-rotation.

A second keys engaged with the key way may be formed on an outer circumferential surface of each of the first and the second moving bodies.

Each of the first and the second legs may be provided to have a plurality of legs.

The first legs may be formed to face the rear of the capsule from the front of the capsule, and the second legs may be formed to face the front of the capsule from the rear of the capsule.

Each of the first and the second moving bodies may include an internal cylinder coupled to the shaft through the hollow portion and an external cylinder coupled to the internal cylinder to surround it. A plurality of leg latching grooves may be formed on the outer circumferential surface of the internal cylinder. A plurality of exposed holes may be formed in the length direction of the capsule on the outer circumferential surface of the external cylinder. The leg latching grooves and the exposed holes may be aligned with each other. The first and the second legs may be disposed at positions corresponding to the positions of the exposed holes of the first and the second moving bodies so as to be hinge-fixed to the external cylinders, respectively. The legs may be unfolded or folded by being respectively latched into the leg latching grooves, depending on the moving direction of the internal cylinder.

The pin latching groove may be formed at the internal cylinder, and the selective moving body may be selectively coupled to or decoupled from the internal cylinders of the first and the second moving bodies.

The pin latching groove may be formed on an inner circumferential surface of the hollow portion in the internal cylinder.

The slits of the capsule and the exposed holes of the external cylinder may be aligned with each other.

A camera may be attached to a head portion of the capsule.

### Advantageous Effects of Invention

A capsule type micro-robot bidirectional moving system can move forward and backward inside internal organs. Since the capsule type micro-robot bidirectional moving system rapidly moves inside internal organs, it can effectively perform the desired photographing or operation.

Further, since the number of actuators for controlling each movement of the system is minimized, the structure of the system is simple, and its energy efficiency is high.

### Brief Description of Drawings

The above and other objects, features and other advantages of the present invention will be more clearly understood from the following detailed description taken in conjunction with the accompanying drawings, in which:
FIG. 1 is an exploded perspective view of a capsule type micro-robot system 100 according to an embodiment;
FIG. 2 is an assembled perspective view of the capsule type micro-robot system 100 according to the embodiment;
FIG. 3 is a perspective view illustrating the first legs 141 respectively exposed to the exterior of a capsule 170 through slits 171;
FIG. 4 is a sectional view taken along line A-A of FIG. 2;
FIG.5 is a conceptual view sequentially illustrating the forward movement of the micro-robot system 100 according to the embodiment;
FIG. 6 is a conceptual view sequentially illustrating the backward movement of the micro-robot system 100 according to the embodiment;
FIG. 7 is a perspective view of a selective moving body 120 according to the embodiment;
FIG. 8 is an exploded view of a first moving body 191 according to the embodiment;
FIG. 9 is a conceptual view illustrating a first latching pin 301 coupled to a pin latching groove 133 of the first moving body 191 according to the embodiment;
FIG. 10 is a conceptual view illustrating the first latching pin 301 decoupled from the pin latching groove 133 of the first moving body 191 according to the embodiment;
FIGS. 11 to 19 are conceptual views sequentially illustrating the principle of coupling and decoupling operations of the selective moving body 120 and the first moving body 191 and the principle of folding and unfolding operations of the first legs 141 according to the embodiment; and
FIG. 20 is a perspective view illustrating the shape of the pin latching groove 133 according to the embodiment.

### Best Mode for Carrying out the Invention

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced items. The use of the terms "first", "second", and the like does not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising" or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skills in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In the drawings, like reference numerals in the drawings denote like elements. The shape, size and regions, and the like, of the drawing may be exaggerated for clarity.

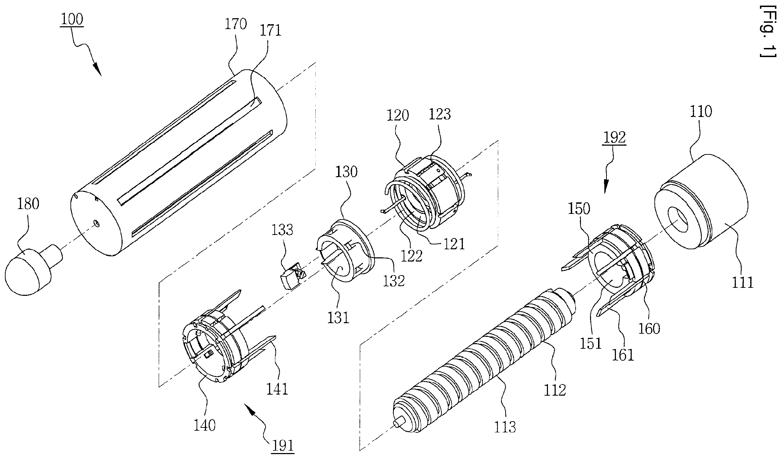

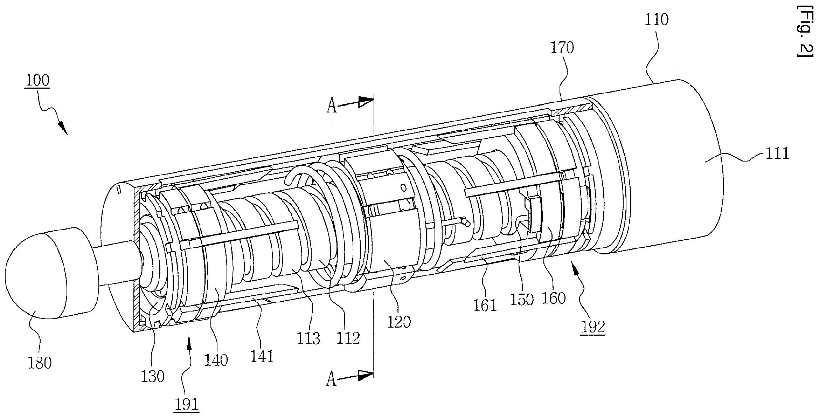

FIG. 1 is an exploded perspective view of a capsule type micro-robot system 100 according to an embodiment. FIG. 2 is an assembled perspective view of the capsule type micro-robot system 100 according to the embodiment. For convenience of illustration, some components are cut away in FIG. 2.

Referring to FIGS. 1 and 2, the capsule type micro-robot system 100 according to this embodiment includes a driving means 110 provided with a shaft 112; a selective moving body 120 coupled to the shaft 112 to linearly move forward and backward along the shaft 112; and a first moving body 191 and a second moving body 192 selectively coupled to or decoupled from the selective moving body 120.

The driving means 110 includes a motor 111, and a shaft 112 connected to the motor 111 and rotated by the motor 111. A screw thread 113 is formed on the outer circumferential surface of the shaft 112.

A hollow portion 121 is formed to pass through the center of the selective moving body 120, and a screw thread 122 engaged with the screw thread 113 of the shaft 112 is formed on the inner circumferential surface of the hollow portion 121. Thus, the shaft 112 is coupled to the selective moving body 120 by passing through the hollow portion 121, and the shaft 112 and the selective moving body 120 are screw-engaged with each other.

The first moving body 191 is disposed at the front of the selective moving body 120. A hollow portion 131 is formed at the center of the first moving body 191, and the first moving body 191 is coupled to the shaft 112 by passing through the hollow portion 131. Unlike the selective moving body 120, a screw thread is not formed on the inner circumferential surface of the hollow portion 131.

Referring to FIG. 1, the first moving body 191 includes an internal cylinder 130 having the hollow portion 131 formed at the center thereof and an external cylinder 140 coupled to the internal cylinder 130 while surrounding the internal cylinder 130. A plurality of first legs 141 are coupled to the external cylinder 140. The first legs 141 are hinge-fixed to the external cylinder 140 so as to be folded or unfolded. The first legs 141 extend in the rear direction from the front thereof so as to be coupled to the external cylinder 140 of the first moving body 191.

The second moving body 192 is disposed at the rear of the selective moving body 120. A hollow portion 151 is formed at the center of the second moving body 192, and the second moving body 192 is coupled to the shaft 112 by passing through the hollow portion 151. A screw thread is not formed on the inner circumferential surface of the hollow portion 151.

Like the first moving body 191, the second moving body 192 includes an internal cylinder 150 having the hollow portion 151 formed at the center thereof and an external cylinder 160 coupled to the internal cylinder 150 while surrounding the internal cylinder 150. A plurality of the second legs 161 are coupled to the external cylinder 160. The second legs 161 are hinge-fixed to the external cylinder 160 so as to be folded or unfolded. The second legs 161 extend in the front direction from the rear thereof so as to be coupled to the external cylinder 160 of the second moving body 192.

According to this embodiment, the structures of the first and the second moving bodies 191 and 192 are identical to each other, and the first and the second moving bodies 191 and 192 are symmetrically disposed at each side of the selective moving body 120.

As illustrated in FIGS. 1 and 2, the micro-robot system 100 further includes a capsule 170 for accommodating the selective moving body 120, the first moving body 191 and the second moving body 192 therein. According to this embodiment, a camera for photographing inside organs is attached to a head portion of the capsule 170.

The capsule 170 is provided with a plurality of slits 171 formed in the length direction of the capsule 170. In this embodiment, the number of the slits 171 is identical to that of each of the first and the second legs 141 and 161.

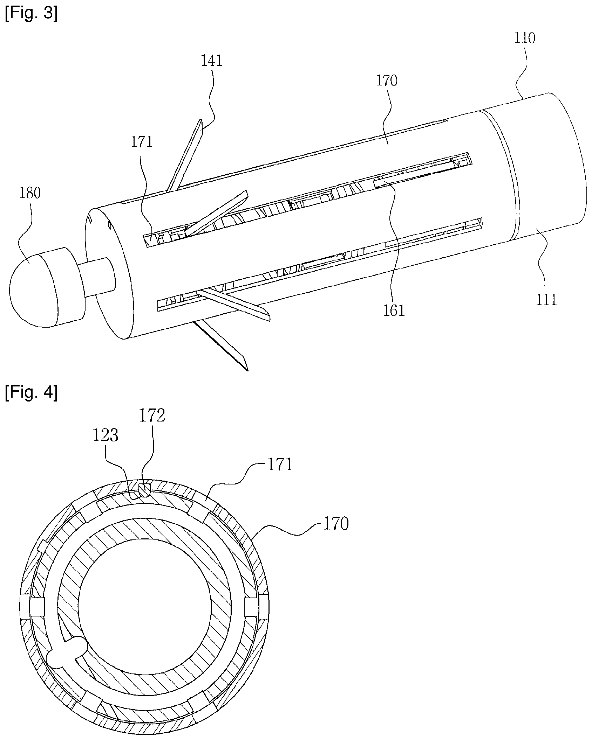

FIG. 3 is a perspective view illustrating the first legs 141 respectively exposed to the exterior of the capsule 170 through the slits 171.

As illustrated in FIG. 3, each of the first and the second legs 141 and 161 are exposed to the exterior of the capsule 170 through the slits 171, respectively, when they are unfolded (see the first legs 141 of FIG. 3), and each of the first and the second legs 141 and 161 are accommodated in the interior of the capsule 170 through the slits 171, respectively, when they are folded (see the second legs 161 of FIG. 3). The first legs 141 are formed so that end portions of the first legs 141 exposed to the exterior of the capsule 170 extend in the rear direction from the rear of the capsule 170. The second legs 161 are formed so that end portions of the second legs 161 exposed to the exterior of the capsule 170 extend in the front direction from the rear of the capsule 170.

According to this embodiment, the selective moving body 120 is screw-engaged with the shaft 112. Therefore, if the shaft 112 is rotated by the motor 111, the selective moving body 120 moves forward and backward in a straight line along the shaft 112. At this time, the rotation of the selective moving body 120 is necessarily controlled so that the selective moving body 120 is not rotated by the rotation of the shaft 112 but moves along the shaft 112.

FIG. 4 is a sectional view taken along line A-A of FIG. 2.

As illustrated in FIG. 4, in order to control the rotation of the selective moving body 120, a key way 172 is formed along the length direction of the capsule 170 on an inner circumferential surface of the capsule 170, and the first key 123 engaged with the key way 172 is formed along the length direction of the selective moving body 120 on an outer circumferential surface of the selective moving body 120. Through the aforementioned structure, the selective moving body 120 does not rotate about the shaft 112 but linearly moves forward and backward while the shaft 112 is rotating.

On the other hand, as described above, a screw thread is not formed on the outer circumferential surface of each of the hollow portions 131 and 151 in the first and the second moving bodies 191 and 192. Therefore, the first and the second moving bodies 191 and 192 are not influenced by the rotation of the shaft 112. That is, if the shaft 112 is rotated by the motor 111, only the selective moving body 120 linearly moves along the shaft 112, and the first and the second moving bodies 191 and 192 do not move under the rotation of the shaft 112. However, the second keys engaged with the key way 172 may be formed in the length directions of the first and the second moving bodies 191 and 192 on the outer circumferential surfaces of the first and the second moving bodies 191 and 192, respectively, so as to prevent the first and the second moving bodies 191 and 192 from being rotated freely on the shaft 112 by a rotation force of the shaft 112 that passes through the centers of the first and the second moving bodies 191 and 192.

According to this embodiment, the selective moving body 120 that moves forward and backward along the shaft 112 is selectively coupled to the first or the second moving body 191 or 192 so that the micro-robot system 100 moves forward and backward. Specifically, when the micro-robot system 100 moves forward, the selective moving body 120 is coupled to the first moving body 191, so that the selective moving body 120 and the first moving body 191 linearly moves together along the shaft 112. When the micro-robot system 100 moves backward, the selective moving body 120 is coupled to the second moving body 192, so that the selective moving body 120 and the second moving body 192 linearly moves together along the shaft 112.

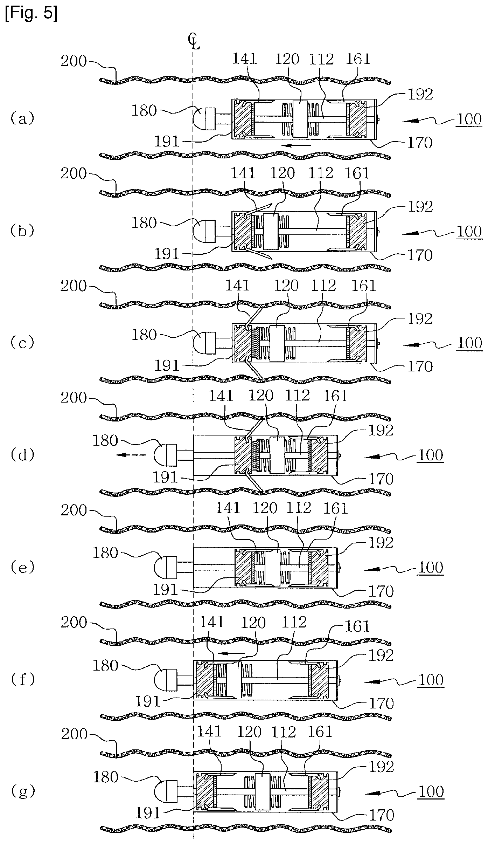

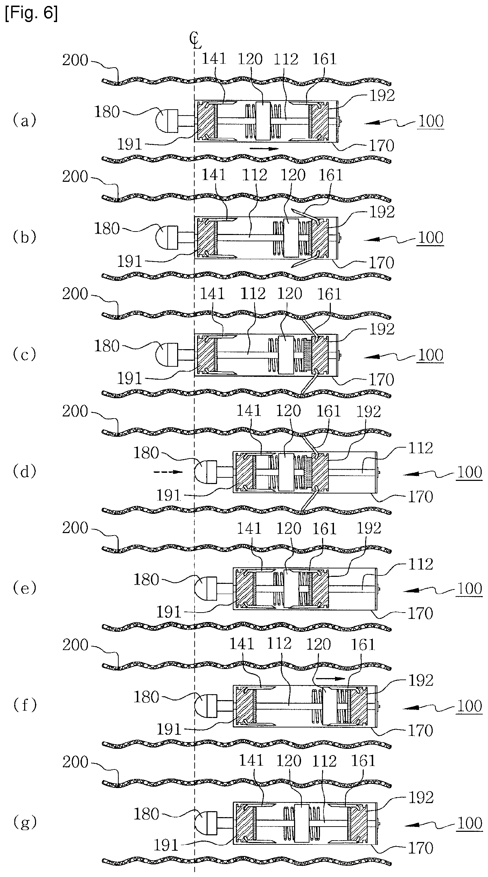

Hereinafter, the principles of the forward and backward movements of the micro-robot system 100 will be described in detail with reference to FIGS. 5 and 6, respectively.

FIG.5 is a conceptual view sequentially illustrating the forward movement of the micro-robot system 100 according to the embodiment. FIG. 6 is a conceptual view sequentially illustrating the backward movement of the micro-robot system 100 according to the embodiment.

First, the principle of the forward movement of the micro-robot system 100 will be described with reference to FIG. 5.

Referring to FIGS. 5a and 5b, the selective moving body 120 moves toward the first moving body 191 (in the direction of the solid line arrow) along the shaft 112 so that the micro-robot system 100 moves forward. If the selective moving body 120 moves and comes in contact with the first moving body 191, it is coupled to the first moving body 191.

Subsequently, as illustrated in FIGS. 5b and 5c, the first legs 141 are unfolded and exposed to the exterior of the capsule 170. The exposed first legs 141 support the inner wall of an internal organ 200 (see FIG. 5c).

In this state, as illustrated in FIG. 5d, the selective moving body 120 moves in the right direction. At this time, the selective moving body 120 and the first moving body 191 are coupled to each other, and the first legs 141 of the first moving body 191 support the inner wall of the internal organ 200. Thus, the selective moving body 120 and the first moving body 191 are in the state that they are kept at their original places, respectively, and the entire capsule 170 is moved forward (in the direction of the dotted line arrow) by the reaction of a force applied to the selective moving body 120. At this time, the position of the selective moving body 120 is controlled so that the rear portion of the selective moving body 120 is not coupled to the second moving body 192.

In the state that the capsule 170 moves forward as illustrated in FIG. 5d, the first legs 141 are folded and accommodated into the capsule 170 as illustrated in FIG. 5f. In the state that the first legs 141 are folded, the selective moving body 120 moves in the left direction (in the direction of the solid line arrow) as illustrated in FIG. 5f. At this time, the first legs 141 are in the sate that they are folded and accommodated into the capsule 170. Therefore, the selective moving body 120 and the first moving body 191 move forward in the left direction without any resistance inside the capsule 170.

When the micro-robot system 100 continuously moves forward inside the internal organ 200, the processes illustrated in FIGS. 5b to 5f are repeatedly performed. When the micro-robot system 100 does not move any more or moves backward, the selective moving body 120 is decoupled from the first moving body 191 as illustrated in FIG. 5g.

Hereinafter, the principle of the backward movement of the micro-robot system 100 will be described with reference to FIG. 6.

Referring to FIGS. 6a and 6b, the selective moving body 120 moves toward the second moving body 192 (in the direction of the solid line arrow) along the shaft 112 so that the micro-robot system 100 moves backward. If the selective moving body 120 moves and comes in contact with the second moving body 192, it is coupled to the second moving body 192.

Subsequently, as illustrated in FIGS. 6b and 6c, the second legs 161 are unfolded and exposed to the exterior of the capsule 170. The exposed second legs 161 support the inner wall of the internal organ 200 (see FIG. 6c).

In this state, as illustrated in FIG. 6d, the selective moving body 120 moves in the right direction. At this time, the selective moving body 120 and the second moving body 192 are coupled to each other, and the second legs 161 of the second moving body 192 support the inner wall of the internal organ 200. Thus, the selective moving body 120 and the second moving body 192 are in the state that they are kept at their original places, respectively, and the entire capsule 170 is moved backward (in the direction of the dotted line arrow) by the reaction of a force applied to the selective moving body 120. At this time, the position of the selective moving body 120 is controlled so that the front portion of the selective moving body 120 is not coupled to the first moving body 191.

In the state that the capsule 170 moves backward as illustrated in FIG. 6d, the second legs 161 are folded and accommodated into the capsule 170 as illustrated in FIG. 6e. In the state that the second legs 161 are folded, the selective moving body 120 moves in the right direction (in the direction of the solid line arrow) as illustrated in FIG. 6f. At this time, the second legs 161 are in the sate that they are folded and accommodated into the capsule 170. Therefore, the selective moving body 120 and the second moving body 192 move backward in the right direction without any resistance inside the capsule 170.

When the micro-robot system 100 continuously moves backward inside the internal organ 200, the processes illustrated in FIGS. 6b to 6f are repeatedly performed. When the micro-robot system 100 does not move any more or moves forward, the selective moving body 120 is decoupled from the second moving body 192 as illustrated in FIG. 6g.

As described above, the micro-robot system 100 according to this embodiment moves forward and backward through the operations of coupling and decoupling the selective moving body 120 to and from the first and the second moving bodies 191 and 192 and the operations of folding and unfolding the first and the second legs 141 and 161. In order to improve the miniaturization and energy efficiency of the system, the micro-robot system 100 according to this embodiment performs the aforementioned coupling/decoupling and folding/unfolding operations using a mechanical link mechanism.

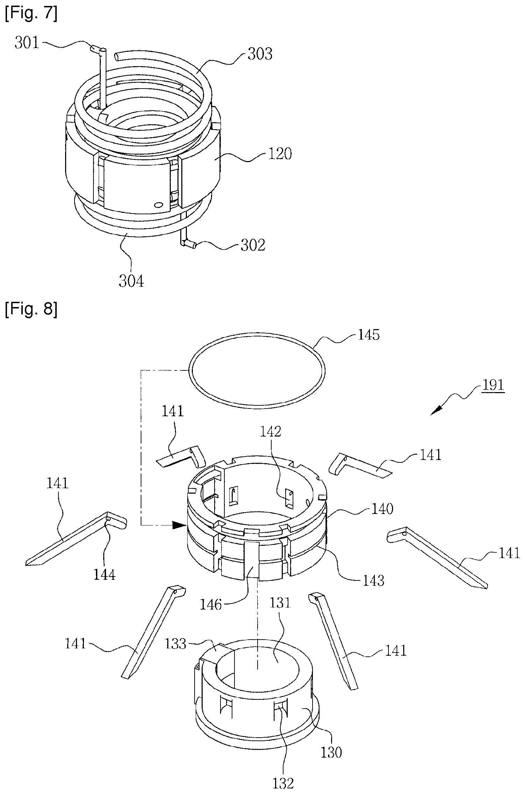

Hereinafter, the configuration for coupling and decoupling the selective moving body 120 to and from the first moving body 191 and the configuration for folding and unfolding the first legs 141 of the first moving body 191 will be described with reference to FIGS. 7 and 8.

FIG. 7 is a perspective view of the selective moving body 120 according to the embodiment.

Referring to FIG. 7, the selective moving body 120 includes a first latching pin 301 formed to extend to the front thereof and a second latching pin 302 formed to extend to the rear thereof. The selective moving body 120 also includes a first elastic body 303 formed at the front thereof and the second elastic body 304 formed at the rear thereof. According to this embodiment, the first and the second elastic bodies 303 and 304 are coil type springs.

FIG. 8 is an exploded view of the first moving body 191 according to the embodiment.

Referring to FIG. 8, a plurality of leg latching grooves 132 are formed on the outer circumferential surface of the internal cylinder 130 in the first moving body 191 along its radial direction. The leg latching grooves 132 function to secure spaces for accommodating portions of the first legs 141, respectively. According to this embodiment, each of the leg latching grooves 132 is formed by digging vertically into the internal cylinder 130 so that its bottom surface is planarized.

A concave groove 146 is formed in the radial direction of each of the first legs 141 on the outer circumferential surface of the external cylinder 140 so that the first legs 141 can be rotated at a predetermined rotating angle. A hinge groove 143 is concavely formed at the outer circumferential surface of the external cylinder 140 so that a wire 145 can be inserted into the hinge groove 143. A exposed hole 142 formed by passing through a portion of the inner surface of each of the concave grooves 146 is formed so that portions of the first legs 141 are accommodated into the leg latching grooves 132 of the internal cylinder 130, respectively. The leg latching grooves 132 and the exposed holes 142 are aligned with each other. The exposed holes 142 are aligned with the slits 171 (see FIG. 1), so that the first legs 141 can be unfolded to the exterior of the capsule 170 through the slits 171 of the capsule 170, respectively.

The wire 145 passes through hinge holes 144 respectively formed at the first legs 141, and portions of the first legs 141 are inserted into the concave grooves 146 to pass through the exposed holes 142 of the external cylinder 140, respectively. Then, the wire 145 is inserted into the hinge groove 143 of the external cylinder 140, so that the plurality of first legs 141 are coupled to the external cylinder 140. Through the aforementioned structure, the first legs 141 are hinge-fixed to the respective concave grooves 146 of the external cylinder 140 so as to be rotated.

The portions of the first legs 141 inserted into the concave grooves 146 by passing through the exposed holes 142 are accommodated in the leg latching grooves 132 of the internal cylinder 130 formed at the center of the external cylinder 140, respectively. Thus, the leg latching grooves 132 respectively allow end portions of the first legs 141 to be latched therein, so that the first legs 141 are folded or unfolded.

Meanwhile, a pin latching groove 133 is formed on an inner circumferential surface of the hollow portion 131 in the internal cylinder 130 (see FIG. 1). The first latching pin 301 of the selective moving body 120 is engaged with the pin latching groove 133 of the first moving body 191.

The first moving body 191 and the selective moving body 120 are coupled to each other by engaging the first latching pin 301 with the pin latching groove 133 of the first moving body 191. The first moving body 191 and the selective moving body 120 are decoupled from each other by decoupling the first latching pin 301 from the pin latching groove 133 of the first moving body 191.

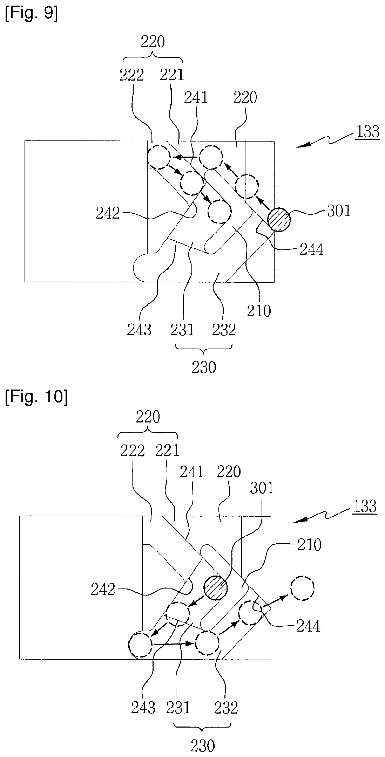

FIG. 9 is a conceptual view illustrating the first latching pin 301 coupled to the pin latching groove 133 of the first moving body 191 according to the embodiment. FIG. 10 is a conceptual view illustrating the first latching pin 301 decoupled from the pin latching groove 133 of the first moving body 191 according to the embodiment.

As illustrated in FIGS. 9 and 10, the pin latching groove 133 includes a coupling portion 210 on which the first latching pin 301 is mounted, an entry portion 220 for forming an entry passage of a pin to the coupling portion 210, and an exit portion 230 for forming an exit passage of the pin.

As illustrated in FIG. 9, when the first moving body 191 and the selective moving body 120 are coupled to each other, the first latching pin 301 is entered into the entry portion and mounted on the coupling portion 210. On the contrary, as illustrated in FIG. 10, when the first moving body 191 and the selective moving body 120 are decoupled from each other, the first latching pin 301 mounted on the coupling portion 210 is exited from the pin latching groove 133 through the exit portion 230.

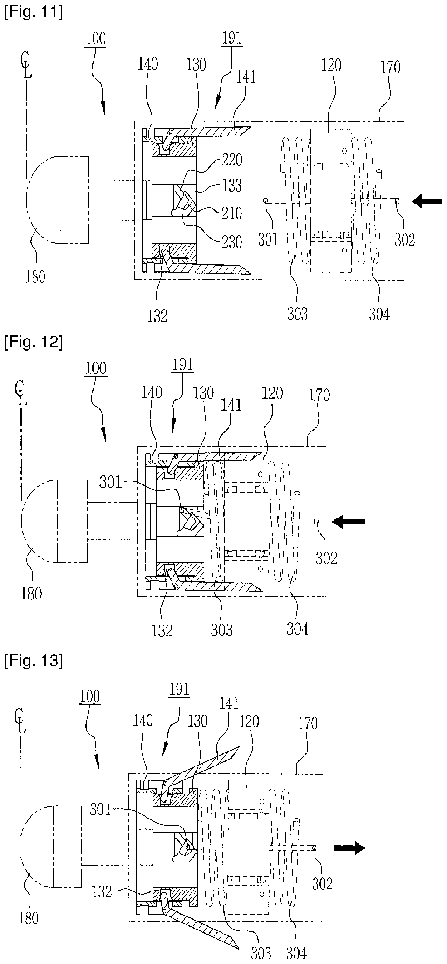

Hereinafter, through the aforementioned configuration, the principle of coupling and decoupling operations of the selective moving body 120 and the first moving body 191 and the principle of folding and unfolding operations of the first legs 141 will be described with reference to FIGS. 11 to 19. FIGS. 11 to 19 are conceptual views sequentially illustrating the principle of the coupling and decoupling operations of the selective moving body 120 and the first moving body 191 and the principle of the folding and unfolding operations of the first legs 141 according to the embodiment.

As illustrated in FIG. 11, the selective moving body 120 is moved toward (left direction) the first moving body 191. As illustrated in FIG. 12, if the selective moving body 120 is moved with the maximum stroke toward (left direction) the first moving body 191, the first elastic body 303 in contact with the rear portion of the first moving body 191 is contracted, and the first latching pin 301 is entered into the pin latching groove 133 through the entry portion 220.

As illustrated in FIG. 13, if the selective moving body 120 is moved in the right direction, the first latching pin 301 is mounted on the coupling portion 210, so that the selective moving body 120 is coupled to the internal cylinder 130 of the first moving body 191. At this time, if the selective moving body 120 is continuously moved in the right direction, the internal cylinder 130 is moved to the right side together with the selective moving body 120. As the internal cylinder 130 is moved to the right side, the upper sidewalls of the leg latching grooves 132 push the end portions of the first legs 141 to the right side, respectively, and the first legs 141 are unfolded by the interference of the leg latching grooves 132.

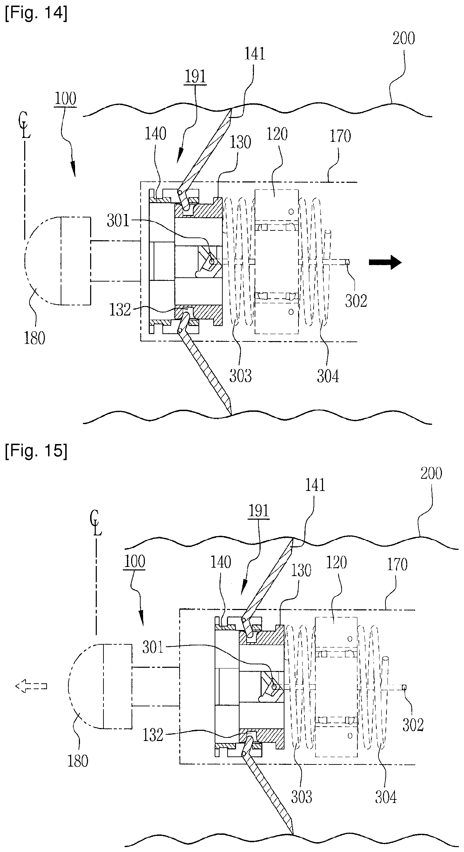

As illustrated in FIG. 14, if the selective moving body 120 is continuously moved in the right direction, the first legs 141 are unfolded at the maximum angle to support the inner wall of the internal organ 200. As illustrated in FIG. 15, if the selective moving body 120 is continuously moved moves in the right direction, the entire capsule 170 moves forward in the left direction (in the direction of the dotted line arrow) (see FIG. 5d).

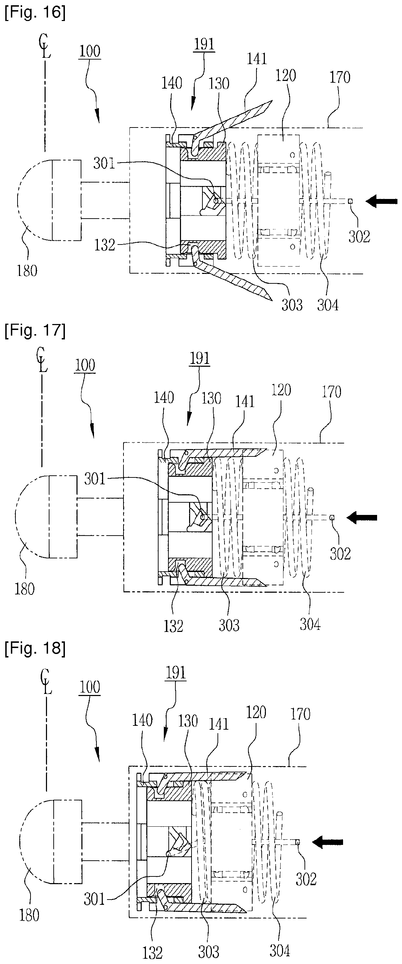

As illustrated in FIG. 16, if the selective moving body 120 is moved to the left direction, the first elastic body 303 supports the internal cylinder 130 so that the first latching pin 301 is not decoupled from the pin latching groove 133. Thus, the internal cylinder 130 is moved in the left direction by the selective moving body 120. As the internal cylinder 130 is moved in the left direction, the lower walls of the leg latching grooves 132 push the first legs 141, respectively, and the first legs 141 start being folded.

As illustrated in FIG. 17, if the selective moving body 120 is continuously moved in the right direction, the first legs 141 are completely folded. If the selective moving body 120 is continuously moved in the left direction in the state that the first legs 141 are completely folded, the internal cylinder 130 pushes the external cylinder 140 so that the external cylinder 140 is moved in the left direction. As a result, the selective moving body 120 and the first moving body 191 are simultaneously moved forward in the left direction within the capsule 170.

As illustrated in FIG. 18, if the selective moving body 120 is continuously moved in the left direction in the state that the selective moving body 120 and the first moving body 191 are moved up to the end of the head portion of the capsule 170, the first elastic body 303 is compressed, and the first latching pin 301 is moved to the exit portion 230.

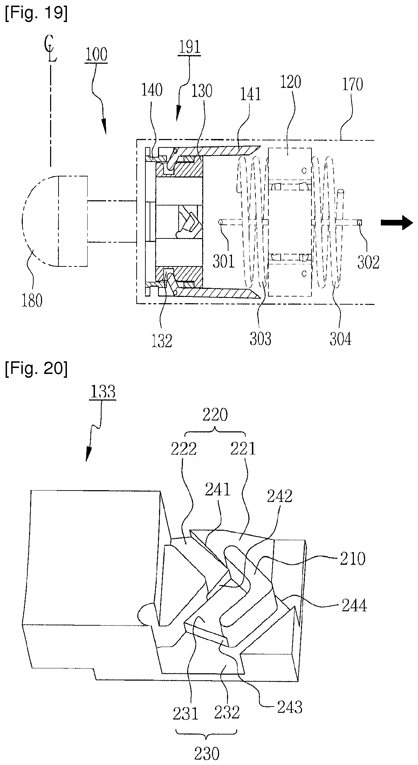

As illustrated in FIG. 19, if the selective moving body 120 is again moved in the right direction, the first latching pin 301 is decoupled from the pin latching groove 133, so that the selective moving body 120 and the first moving body 191 are decoupled from each other.

As described above, the selective moving body 120 and the first moving body 191 are coupled to or decoupled from each other as the first latching pin 301 and the pin latching groove 133 are coupled to or decoupled from each other. Therefore, in order to operate the system under a desired control, it is required to allow the coupling and decoupling operations of the first latching pin 301 and the pin latching groove 133 to be performed properly.

According to this embodiment, in order to allow the coupling and decoupling operations of the first latching pin 301 and the pin latching groove 133 to be performed properly, the pin latching groove 133 is formed so that the first latching pin 301 can be mounted on the coupling portion 210 by allowing the first latching pin 301 to enter into the pin latching groove 133 only through the entry portion 220 and so that the first latching pin 301 can be exited from the pin latching groove 133 only through the exit portion 230.

Specifically, the pin latching groove 133 according to this embodiment is formed to have multi-layered steps. FIG. 20 is a perspective view illustrating the shape of the pin latching groove 133 according to the embodiment.

Referring to FIG. 20, the entry portion 220 includes a first entry path 221 for forming an entry path of the first latching pin 301 to the pin latching groove 133 and a second entry path 222 for forming an entry path of the first latching pin 301 to the coupling portion 210. The exit portion 230 includes a first exit path 231 for forming an exit path of the first latching pin 301 from the coupling portion 220 and a second exit path 232 for forming an exit path of the first latching pin 301 from the pin latching groove 133.

As illustrated in FIG. 20, a step 241 is formed at the boundary between the first and the second exit paths 221 and 222, and a step 242 is also formed at the boundary between the second exit path 222 and the first exit path 231. A step 243 is formed at the boundary between the first and the second exit paths 231 and 232, and a step 244 is also formed at the boundary between the second exit path 232 and the first entry path 221.

Referring back to FIGS. 9 and 10, the first latching pin 301 entering into the pin latching grooves 133 does not enter through the second exit path 232 due to the step 244 but enters through the first entry path 221. The first latching pin 301 entering into the second entry path 222 does not go backward to the first entry path 221 due to the step 241 but is mounted on the coupling portion 210 along the second entry path 222 (see FIGS. 12 and 13). Through the same principle, the first latching pin 301 mounted on the coupling portion 210 does not go backward along the second entry path 222 but is decoupled from the pin latching groove 133 by sequentially passing through the first and the second exit paths 231 and 232.

According to this embodiment, in order to allow the first latching pin 301 to be moved along the pin latching groove 133 having the steps formed as described above, the first latching pin 301 has elasticity so that its end portion can be bent up and down or left and right. However, since the first latching pin 301 has a constant hardness, the coupling relation between the selective moving body 120 and the first moving body 191 can be strongly maintained in the state that the first latching pin 301 is mounted on the coupling portion 210.

As described above, the configurations and principles have been described, in which the selective moving body 120 and the first moving body 191 are coupled to or decoupled from each other and the first legs 141 are folded or unfolded.

In this embodiment, the first and the second moving bodies 191 and 192 have the same configuration. It will be readily understood that the coupling/decoupling of the second latching pin 302 of the selective moving body 120 and the pin latching groove 133 formed at the second moving body 192 and the configuration and operation for folding/unfolding the second legs 161 of the second moving body 192 are performed through the same principles as described above. Therefore, their detailed descriptions will be omitted.

Although the preferred embodiments of the present invention have been disclosed for illustrative purposes, those skilled in the art will appreciate that various modifications, additions and substitutions are possible, without departing from the scope of the invention as defined in the accompanying claims.

## Claims

1. A capsule type micro-robot bidirectional moving system (100), the system comprising:
a selective moving body (120) adapted to linearly move forward and backward along a predetermined axis;
a driving means (110) adapted to move the selective moving body;
a first moving body (191) disposed at the front of the selective moving body;
a second moving body (192) disposed at the rear of the selective moving body; first legs (141) coupled to the first moving body so as to be folded or unfolded; and second legs (161) coupled to the second moving body so as to be folded or unfolded, wherein the selective moving body is adapted to selectively couple to or decouple from the first or second moving body.

2. The system according to claim 1, wherein:
when the system moves forward, the selective moving body is coupled to the first moving body to allow the first moving body to linearly move along the axis; and
when the system moves backward, the selective moving body is coupled to the second moving body to allow the second moving body to linearly move along the axis.

3. The system according to claim 2, wherein:
the selective moving body comprises a first latching pin formed to extend to the front thereof and a second latching pin formed to extend to the rear thereof; and
pin latching grooves having the first and the second latching pins coupled or decoupled thereto or therefrom are formed at the first and the second moving bodies, respectively.

4. The system according to claim 3, wherein:
the pin latching groove comprises a coupling portion having the latching pin mounted thereon, an entry portion for forming an entry path to the coupling portion, and an exit portion for forming an exit path from the coupling portion; and
the latching pin is coupled to the pin latching groove by being entered into the entry portion and mounted on the coupling portion, and the latching pin mounted on the coupling portion is decoupled from the pin latching groove by being exited through the exit portion.

5. The system according to claim 4, wherein steps are formed at the boundaries between the entry portion, the coupling portion and the exit portion, respectively.

6. The system according to claim 5, wherein:
the entry portion comprises a first entry path for forming a path through which the latching pin enters into the pin latching groove and a second entry path for forming a path through which the latching pin enters into the coupling portion;
the exit portion comprises a first exit path for forming a path through which the latching pin is decoupled from the coupling portion and a second exit path for forming a path through which the latching pin is decoupled from the pin latching groove; and
steps are formed at the boundary between the first and the second entry paths and at the boundary between the first and the second exit paths, respectively.

7. The system according to claim 6, wherein the selective moving body comprises a first elastic body formed at the front thereof and a second elastic body formed at the rear thereof.

8. The system according to claim 7, wherein the first and second elastic bodies are coil type springs.

9. The system according to claim 8, further comprising a capsule for accommodating the selective moving body, the first moving body and the second moving body therein, wherein:
the capsule is provided with slits formed along the length direction thereof; and
each of the first and the second legs are respectively exposed to the exterior of the capsule through the slits when they are unfolded, and each of the first and the second legs are respectively accommodated in the interior of the capsule through the slits when they are folded.

10. The system according to claim 9, wherein:
the driving means comprises a shaft formed along the length direction of the capsule to be rotated by a motor;
hollow portions are formed at the center of the selective moving body, the first moving body and the second moving body so as to pass through them, respectively; and
the shaft is coupled to the selective moving body, the first moving body and the second moving body by passing through them.

11. The system according to claim 10, wherein:
a screw thread is formed on the outer circumferential surface of the shaft;
a screw thread engaged with the screw thread of the shaft is formed on the inner circumferential surface of the hollow portion in the selective moving body; and
the shaft and the selective moving body are screw-engaged with each other.

12. The system according to claim 11, wherein a first key is formed along the length direction of the capsule on an outer circumferential surface of the selective moving body, and a key way engaged with the first key is formed on an inner circumferential surface of the capsule,
wherein a second key engaged with the key way is formed on an outer circumferential surface of each of the first and the second moving bodies.

13. The system according to claim 12, wherein each of the first and the second legs is provided to have a plurality of legs,
wherein the first legs are formed to face the rear of the capsule from the front of the capsule, and the second legs are formed to face the front of the capsule from the rear of the capsule.

14. The system according to claim 13, wherein:
each of the first and the second moving bodies comprises an internal cylinder coupled to the shaft through the hollow portion and an external cylinder coupled to the internal cylinder to surround it;
a plurality of leg latching grooves are formed on the outer circumferential surface of the internal cylinder;
a plurality of exposed holes are formed along the length direction of the capsule on the outer circumferential surface of the external cylinder;
the leg latching grooves and the exposed holes are aligned with each other;
the first and the second legs are disposed at positions corresponding to the positions of the exposed holes of the first and the second moving bodies so as to be hinge-fixed to the external cylinders, respectively; and
the legs are unfolded or folded by being respectively latched into the leg latching grooves, depending on the moving direction of the internal cylinder.

15. The system according to claim 14, wherein the pin latching groove is formed at the internal cylinder, and the selective moving body is selectively coupled to or decoupled from the internal cylinders of the first and the second moving bodies, wherein the pin latching groove is formed on an inner circumferential surface of the hollow portion in the internal cylinder.

## Patentansprüche

1. Kapselartiges mikrorobotisches bidirektional bewegliches System (100), umfassend:
einen selektiv beweglichen Körper (120), der zur linearen Vorwärts- und Rückwärtsbewegung entlang einer vorbestimmten Achse ausgebildet ist;
eine Antriebseinrichtung (110), die zur Bewegung des selektiv beweglichen Körpers ausgebildet ist;
einen ersten beweglichen Körper (191), der an der Vorderseite des selektiv beweglichen Körpers angeordnet ist;
einen zweiten beweglichen Körper (192), der an der Rückseite des selektiv beweglichen Körpers angeordnet ist;
erste Beine (141), die ein- und ausklappbar an den ersten beweglichen Körper angelenkt sind; und
zweite Beine (161), die ein- und ausklappbar an den zweiten beweglichen Körper angelenkt sind,
wobei der selektiv bewegliche Körper zur selektiven Kopplung oder Abkopplung an bzw. von dem ersten oder dem zweiten beweglichen Körper ausgebildet ist.

2. System nach Anspruch 1, wobei:
der selektiv bewegliche Körper bei Vorwärtsbewegung des Systems an den ersten beweglichen Körper gekoppelt ist, so dass eine lineare Bewegung des ersten beweglichen Körpers entlang der Achse ermöglicht ist; und
der selektiv bewegliche Körper bei Rückwärtsbewegung des Systems an den zweiten beweglichen Körper gekoppelt ist, so dass eine lineare Bewegung des zweiten beweglichen Körpers entlang der Achse ermöglicht ist.

3. System nach Anspruch 2, wobei:
der selektiv bewegliche Körper einen sich zur Vorderseite des selektiv beweglichen Körpers erstreckend ausgebildeten ersten Raststift und einen sich zur Rückseite des selektiv beweglichen Körpers erstreckend ausgebildeten zweiten Raststift umfasst; und
an dem ersten und an dem zweiten beweglichen Körper Stiftrastnuten ausgebildet sind, an welche bzw. von welchen der erste Raststift bzw. der zweite Raststift koppelbar oder abkoppelbar ist.

4. System nach Anspruch 3, wobei:
die Stiftrastnut einen Koppelabschnitt, an welchem der Raststift angebracht ist, einen Eintrittsabschnitt zur Ausbildung eines Eintrittswegs in den Koppelabschnitt und einen Austrittsabschnitt zur Ausbildung eines Austrittsweg aus dem Koppelabschnitt umfasst; und
der Raststift durch Einführen des Raststifts in den Eintrittsabschnitt und Anbringen an dem Koppelabschnitt mit der Stiftrastnut koppelbar ist und der an dem Koppelabschnitt angebrachte Raststift durch Herausführen des Raststifts durch den Austrittsabschnitt von der Stiftrastnut abkoppelbar ist.

5. System nach Anspruch 4, wobei an den Übergängen zwischen dem Eintrittsabschnitt, dem Koppelabschnitt und dem Austrittsabschnitt jeweils Stufen ausgebildet sind.

6. System nach Anspruch 5, wobei:
der Eintrittsabschnitt einen ersten Eintrittsweg zur Ausbildung eines Wegs, durch welchen der Raststift in die Stiftrastnut eintritt, und einen zweiten Eintrittsweg zur Ausbildung eines Wegs, durch welchen der Raststift in den Koppelabschnitt eintritt, umfasst;
der Austrittsabschnitt einen ersten Austrittsweg zur Ausbildung eines Wegs, durch welchen der Raststift von dem Koppelabschnitt abkoppelbar ist, und einen zweiten Austrittsweg zur Ausbildung eines Wegs, durch welchen der Raststift von der Stiftrastnut abkoppelbar ist, umfasst; und
an dem Übergang zwischen dem ersten und dem zweiten Eintrittsweg und an dem Übergang zwischen dem ersten und dem zweiten Austrittsweg jeweils Stufen ausgebildet sind.

7. System nach Anspruch 6, wobei der selektiv bewegliche Körper einen an der Vorderseite desselben ausgebildeten ersten elastischen Körper und einen an der Rückseite desselben ausgebildeten zweiten elastischen Körper umfasst.

8. System nach Anspruch 7, wobei der erste und der zweite elastische Körper Schraubenfedern sind.

9. System nach Anspruch 8, des Weiteren umfassend eine Kapsel zur Aufnahme des selektiv beweglichen Körpers, des ersten beweglichen Körpers und des zweiten beweglichen Körpers in deren Innerem, wobei:
die Kapsel mit entlang der Längsrichtung derselben ausgebildeten Schlitzen versehen ist; und die ersten und die zweiten Beine im ausgeklappten Zustand jeweils durch die Schlitze gegenüber dem Äußeren der Kapsel freiliegen und die ersten und die zweiten Beine im eingeklappten Zustand jeweils durch die Schlitze im Inneren der Kapsel aufgenommen sind.

10. System nach Anspruch 9, wobei:
die Antriebseinrichtung eine entlang der Längsrichtung der Kapsel ausgebildete, durch einen Motor drehbare Welle umfasst;
in der Mitte des selektiv beweglichen Körpers, des ersten beweglichen Körpers und des zweiten Beweglichen Körpers jeweils diese durchgreifende hohle Abschnitte ausgebildet sind; und
die Welle mit dem selektiv beweglichen Körper, dem ersten beweglichen Körper und dem zweiten Beweglichen Körper gekoppelt ist, indem sie diese durchgreift.

11. System nach Anspruch 10, wobei:
ein Gewinde an der Außenumfangsfläche der Welle ausgebildet ist;
ein mit dem Gewinde der Welle in Eingriff stehendes Gewinde an der Innenumfangsfläche des hohlen Abschnitts in dem selektiv beweglichen Körper ausgebildet ist; und
die Welle und der selektiv bewegliche Körper miteinander in Gewindeeingriff stehen.

12. System nach Anspruch 11, wobei ein erster Keil entlang der Längsrichtung der Kapsel an einer Außenumfangsfläche des selektiv beweglichen Körpers ausgebildet ist und eine mit dem ersten Keil in Eingriff stehende Keilnut an einer Innenumfangsfläche der Kapsel ausgebildet ist,
wobei an einer Außenumfangsfläche des ersten und des zweiten beweglichen Körpers jeweils ein mit der Keilnut in Eingriff stehender zweiter Keil ausgebildet ist.

13. System nach Anspruch 12, wobei die ersten und die zweiten Beine jeweils eine Vielzahl von Beinen umfassen,
wobei die ersten Beine von der Vorderseite der Kapsel zur Rückseite der Kapsel weisend ausgebildet sind und die zweiten Beine von der Rückseite der Kapsel zur Vorderseite der Kapsel weisend ausgebildet sind.

14. System nach Anspruch 13, wobei:
der erste und der zweite bewegliche Körper jeweils einen durch den hohlen Abschnitt mit der Welle gekoppelten Innenzylinder und einen den Innenzylinder umgebend mit dem Innenzylinder gekoppelten Außenzylinder umfassen;
eine Vielzahl von Beinrastnuten an der Außenumfangsfläche des Innenzylinders ausgebildet ist;
eine Vielzahl von freiliegenden Löchern entlang der Längsrichtung der Kapsel an der Außenumfangsfläche des Außenzylinders ausgebildet ist;
die Beinrastnuten und die freiliegenden Löcher miteinander fluchten;
die ersten und die zweiten Beine zur Anlenkung an den Außenzylindern an Stellen angeordnet sind, welche den Stellen der freiliegenden Löcher des ersten bzw. zweiten beweglichen Körpers entsprechen; und
die Beine je nach Bewegungsrichtung des Innenzylinders jeweils durch Einrasten in die Beinrastnuten aus- bzw. einklappbar sind.

15. System nach Anspruch 14, wobei die Stiftrastnut an dem Innenzylinder ausgebildet ist und der selektiv bewegliche Körper selektiv an die Innenzylinder des ersten und des zweiten beweglichen Körpers ankoppelbar bzw. von diesen abkoppelbar ist, wobei die Stiftrastnut an einer Innenumfangsfläche des hohlen Abschnitts in dem Innenzylinder ausgebildet ist.

## Revendications

1. Système (100) mobile bidirectionnel de micro-robot de type capsule, le système comprenant :
un corps sélectivement mobile (120) destiné à se déplacer linéairement en avant et en arrière le long d'un axe prédéterminé ;
un moyen d'entraînement (110) destiné à déplacer le corps sélectivement mobile ;
un premier corps mobile (191) disposé à l'avant du corps sélectivement mobile ;
un deuxième corps mobile (192) disposé à l'arrière du corps sélectivement mobile ;
des premiers bras (141) couplés au premier corps mobile de manière à être pliés ou dépliés ; et
des deuxièmes bras (161) couplés au deuxième corps mobile de manière à être pliés ou dépliés,
dans lequel le corps sélectivement mobile est destiné à être sélectivement couplé au ou découplé du premier ou deuxième corps mobile.

2. Système selon la revendication 1, dans lequel :
quand le système se déplace en avant, le corps sélectivement mobile est couplé au premier corps mobile afin de permettre au premier corps mobile de se déplacer linéairement le long de l'axe ; et
quand le système se déplace en arrière, le corps sélectivement mobile est couplée au deuxième corps mobile afin de permettre au deuxième corps mobile de se déplacer linéairement le long de l'axe.

3. Système selon la revendication 2, dans lequel :
le corps sélectivement mobile comprend une première tige de verrouillage formée de manière à s'étendre vers l'avant de celui-ci et une deuxième tige de verrouillage formée de manière à s'étendre vers l'arrière de celui-ci ; et
des rainures de verrouillage de tige ayant la première et la deuxième tige de verrouillage couplées à celles-ci ou découplées de celles-ci sont respectivement formées sur le premier et le deuxième corps mobile.

4. Système selon la revendication 3, dans lequel :
la rainure de verrouillage de tige comprend une partie de couplage ayant la tige de verrouillage montée sur celle-ci, une partie d'entrée destinée à former une voie d'entrée de la partie de couplage, et une partie de sortie destinée à former une voie de sortie de la partie de couplage ; et
la tige de verrouillage est couplée à la rainure de verrouillage de tige en étant introduite dans la partie d'entrée et montée sur la partie de couplage, et la tige de verrouillage montée sur la partie de couplage est découplée de la rainure de verrouillage de tige en étant sortie par la the partie de sortie.

5. Système selon la revendication 4, dans lequel des gradins sont respectivement formés aux jonctions entre la partie d'entrée, la partie de couplage et la partie de sortie.

6. Système selon la revendication 5, dans lequel :
la partie d'entrée comprend une première voie d'entrée destinée à former une voie par laquelle la tige de verrouillage entre dans la rainure de verrouillage de tige et une deuxième voie d'entrée destinée à former une voie par laquelle la tige de verrouillage entre dans la partie de couplage ;
la partie de sortie comprend une première voie de sortie destinée à former une voie par laquelle la tige de verrouillage est découplée de la partie de couplage et une deuxième voie de sortie destinée à former une voie par laquelle la tige de verrouillage est découplée de la rainure de verrouillage de tige ; et
des gradins sont respectivement formés à la jonction entre la première et la deuxième voie d'entrée et à la jonction entre la première et la deuxième voie de sortie.

7. Système selon la revendication 6, dans lequel le corps sélectivement mobile comprend un premier corps élastique formé à l'avant de celui-ci et un deuxième corps élastique formé à l'arrière de celui-ci.

8. Système selon la revendication 7, dans lequel le premier corps élastique et le deuxième corps élastique sont des ressorts hélicoïdaux.

9. Système selon la revendication 8, comprenant en outre une capsule destiné à loger le corps sélectivement mobile, le premier corps mobile et le deuxième corps mobile dans celle-ci, dans lequel :
la capsule est munie de fentes formées le long de la direction longitudinale de celle-ci ; et
chacun des premiers et deuxièmes bras est exposé à l'extérieure de la capsule par les fentes quand ils sont dépliés, et chacun des premiers et deuxièmes bras est respectivement logé dans l'intérieur de la capsule par les fentes quand ils sont pliés.

10. Système selon la revendication 9, dans lequel :
le moyen d'entrainement comprend un arbre formé le long de la direction longitudinale de la capsule de manière à être tourné par un moteur ;
des parties creuses sont formées au centre du corps sélectivement mobile, du premier corps mobile et du deuxième corps mobile de manière à traverser ceux-ci; et
l'arbre est couplé au corps sélectivement mobile, au premier corps mobile et au deuxième corps mobile en traversant ceux-ci.

11. Système selon la revendication 10, dans lequel :
un filetage est formé sur la surface circonférentielle extérieure de l'arbre ;
un filetage engagé avec le filetage de l'arbre est formé sur la surface circonférentielle intérieure de la partie creuse dans le corps sélectivement mobile ; et
l'arbre et le corps sélectivement mobile sont engagés l'un avec l'autre par filetage.

12. Système selon la revendication 11, dans lequel une première clavette est formée le long de la direction longitudinale de la capsule sur une surface circonférentielle extérieure du corps sélectivement mobile, et une rainure de clavette engagée avec la première clavette est formée sur une surface circonférentielle intérieure de la capsule,
dans lequel une deuxième clavette engagée avec la rainure de clavette est formée sur une surface circonférentielle extérieure de chacun des premier et deuxième corps mobiles.

13. Système selon la revendication 12, dans lequel chacun des premiers et deuxièmes bras comprend une pluralité de bras,
dans lequel les premiers bras sont formés de manière à être orientés vers l'arrière de la capsule à partir du devant de la capsule, et les deuxièmes bras sont formés de manière à être orientés vers le devant de la capsule à partir de l'arrière de la capsule.

14. Système selon la revendication 13, dans lequel :
chacun des premier et deuxième corps mobiles comprend un cylindre interne couplé à l'arbre par la partie creuse et un cylindre externe couplé au cylindre interne de manière à entourer celui-ci ;
une pluralité de rainures de verrouillage de bras sont formées sur la surface circonférentielle extérieure du cylindre interne ;
une pluralité de trous exposés sont formés le long de la direction longitudinale de la capsule sur la surface circonférentielle extérieure du cylindre externe ;
les rainures de verrouillage de bras et les trous exposés sont alignés entre eux ;
les premiers et deuxièmes bras sont disposés respectivement à des positions correspondant aux positions des trous exposés des premier et deuxième corps mobiles afin d'être articulés aux cylindres externes ; et
les bras sont dépliés ou pliés en étant respectivement verrouillés dans les rainures de verrouillage de bras en fonction de à la direction de déplacement du cylindre interne.

15. Système selon la revendication 14, dans lequel la rainure de verrouillage de tige est formée sur le cylindre interne, et le corps sélectivement mobile est sélectivement couplé aux ou découplé des cylindres internes des premier et deuxième corps mobiles,
dans lequel la rainure de verrouillage de tige est formée sur une surface circonférentielle intérieure de la partie creuse dans le cylindre interne.
